# EUROPEAN PATENT SPECIFICATION

(11) **EP 1 820 848 B1**
(45) Date of publication and mention of the grant of the patent: **02.12.2009**
(21) Application number: 05811882.9
(22) Date of filing: 04.11.2005
(51) Int. Cl.: C12N 1/20, C12P 7/26, C07C 49/17, C12R 1/07

(54) **AN ACETOIN HIGH YIELD BACILLUS PUMILUS STRAIN**
BACILLUS PUMILUS-STAMM MIT HOHER ACETOINPRODUKTION
SOUCHE DE BACILLUS PUMILUS A HAUT RENDEMENT D'ACETOINE

(30) Priority: 19.11.2004 CN 200410084381
(43) Date of publication of application: 22.08.2007
(73) Proprietor: Shanghai Apple Flavor&Fragrance Co. Ltd., Jiading District Shanghai 201809 (CN)
(72) Inventor: XU, Ping, Shanghai 201809 (CN); XIAO, Zijun, Shanghai 201809 (CN); DU, Yi, Shanghai 201809 (CN); WEI, Zhonghao, Shanghai 201809 (CN)
(74) Representative: Gevers, François
(86) International application number: PCT/CN2005/001849
(87) International publication number: WO 2006/053480

(56) References cited:
- EP-A2- 0 128 714
- EP-A2- 0 372 332
- EP-A2- 0 430 406
- RYU CHOONG-MIN ET AL: "Bacterial volatiles promote growth in Arabidopsis." PROCEEDINGS OF THE NATIONAL ACADEMY OF SCIENCES OF THE UNITED STATES OF AMERICA, vol. 100, no. 8, 15 April 2003 (2003-04-15), pages 4927-4932, XP007903205 ISSN: 0027-8424

## Description

### FIELD OF THE INVENTION

This invention belongs to the domain of biotechnology, and relates to an isolated bacterial strain for the production of high-yield of acetoin (see EP 430 406).

### BACKGROUND OF THE INVENTION

Acetoin is a favorable flavor and it is widely used in the world as spicery to concoct the fragrances of cream, yoghourt, strawberry and so on. Acetoin has a pleasing aroma of cream. It is usually used as the intensifier of the perfume of cream, cheese, coffee, nut, etc. Acetoin can also change the flavor of beer and cheese during their fermentation. Nowadays the consumption of milk products are growing and more and more people prefer foods of milky taste. The study on acetoin production has aroused the attention of companies and research institutions in the world (see for example EP128714 or Ryu Choong-Min et al. "Bacterial Volatiles promote growth in Arabidopsis" Proceedings of the National Academy of sciences of the USA, vol 100, No.8,15 April 2003 - pp 4927-4932).

At present, the preparation methods of acetoin in laboratories mainly include: extracting from plant materials containing acetoin, biological methods, oxidating 2,3-butanedione using catalyzers, oxidating butanone using electrochemical methods, hydrolyzing linear ketone in dilute sulfuric acid using thallium salt, transformation of butanedione or 2,3-butanediol, etc.

The studies on acetoin production were first reported in the early 20st century. The methods employed were partial deoxidation of 2,3-butanedione using zinc and acids, and selective oxidation of 2,3-butanediol. Recently, many biological techniques on manufacturing acetoin have been reported. For example, acting on 2,3-butanediol by mycoderma, or acting on sugarcane juice by aspergillus, penicillium or other epiphytes. But these studies were conducted in lab settings. To meet the needs of environmentalism and green chemical industries, these biological methods will be the main research fields in the future.

The main industrial methods to produce acetoin are the chemosynthesis methods using 2,3-butanedione as the substrate. In 1989, Ehime University of Japan succeeded in obtaining acetoin by reducing 2,3-butanedione in the system of Zn-ZnCl-EtOH. In this method, the reaction was carried out by heating and circumfluence at natural pressure, and acetoin was obtained after further separation and purification. The circumfluence temperature was about 70°C ∼ 80°C, while the recovery efficiency of the product was 71%. In 1992, Hangzhou University of China developed a new reducing method to produce acetoin with NaHSe. In this method, selenium powder was added into the NaHB solution in a stirring reactor. After NaHSe was formed under vacuum, the mixed solution of acetic acid, ethanol and 2,3-butanedione dissolved in tetrahydrofuran were added into the stirring reactor at room temperature. The yield was 57% using this method.

In 1998, Martin Studer et al. of Witwatersrand University used platinum denaturalized by 10,11-dihydrgen cinchona ledgeriana(HCD) as catalyst to selectively deoxidize 2,3-butanedione by hydrogenation. Butanedione, catalyst and HCD in toluene were added into a high-pressure reactor. The reaction pressure was 10.7 Mpa, while the temperature was 0°C ∼ 25°C. The reaction was stopped after about 10 min. The recovery efficiency of the product was 85%, and some optically active byproducts were also obtained. This reaction was a catalytic hydrogenation, and the control of the reaction conditions was the key, because acetoin would be further converted to 2,3-butanediol if the reaction continued, and the recovery efficiency of the product only amounted to 50%. Slipszenko et al. of Hull University also performed the research on butanedione deoxidation by selective catalytic hydrogenation using platinum as the catalyst. But the solvent used was methylene dichloride. The reaction pressure, temperature, and yield were 1 Mpa, 5°C ∼ 25°C, and 85%, respectively. In this method, the (R)-acetoin enantiomer could be over produced by controlling the reaction time and the partial pressure of hydrogen, yielding 70%.

The reaction of catalytic hydrogenation was started at high pressure, demanding specific equipments. The catalyst used in the reaction was a kind of precious metals, which was costly, and some problems concerning the catalyst such as its manufacture, denaturalization, poisoning, and regeneration have not been solved, which confined the method only in laboratory study.

In 1992, enzymes from microorganisms were used as catalysts for acetoin production by Hummel et al. from the United States. In this method, the butanedione reductases from lactic bacteria and saccharomycetes were obtained to convert butanedione to acetoin at the presence of NADPH at pH 5 and 70°C, yielding 100%. Because the catalytic activities of some enzymes are stereospecific, this method was good at chiral compound production, yielding no or minor isomers. The advantages using reductases, such as high-selective, high-yield, and safe in food-additive production, are obvious. But the key step of this method is how to obtain the butanedione reductases. It is a very important study field in the days of green chemical industry.

The project of producing acetoin using fermentation technology is an important aspect of biological processes. The metabolic pathway of acetoin production using glucose or other substrates has been elaborated (Figure i), which provides the theory for the production of acetoin by fermentation. Although there were some reports including a few patents on this method, most of them were still restricted in laboratories. It is an important step to isolate a high-yield acetoin-producing strain for the production of acetoin by microbial fermentation. So far, the reported strains for acetoin production included *Klebsiella pneumoniae*, *Klebsiella oxytoca, Aeromonas hydrophilia, Bacillus subtilis, Bacillus polymyxa, Bacillus licheniformis, Serratia marcescens, Listeria monocytogenes, Aerobacter aerogenes, Bacillus amyloliquefaciens, Enterobacter aerogenes, Lactococcus lactis, Lactobacillus casei, Streptococcus thermophilus, Leuconoctoc mesenteroides, Leuconoctoc lactis, Leuconoctoc oenos, Leuconoctoc pseudomesenteroides, Bacillus stearothermophilus, Hanseniaspora guillieromondii, Saccharomyces carlsbergensis, Saccharomycodes ludwigii, Zygosaccharomyces bailli, Zygosaccharomyces fermentati* and so on. However, there were still some problems remained when employing the strains above to produce acetoin. That is, the yield of acetoin was too low and acetoin was produced only as the by-product of 2, 3-butanediol fermentation. Therefore, all the strains above could hardly be employed in industrial acetoin fermentation.

Due to the low concentration of acetoin in plants, extraction of acetoin from plant materials is a costly technology and unfit for industrialization. While the method of chemical synthesis can bring in high yield of acetoin, it demands intensive reaction conditions and more sophisticated equipments. Moreover, the resulting acetoin is not regarded as green product and there are serious problem in protecting environment. In contrast, the method of biosynthesis, including fermentation with microorganisms, is only studied in the laboratory-scale, mostly because of problems that lead to the low concentration of the product such as the strain, enzyme activity, optimization of fermentation conditions and process controls.

### SUMMARY OF THE INVENTION

Due to the current difficulties and problems with acetoin production, the aim of this invention is to supply a newly isolated high-yield *Bacillus pumilus* for producing acetoin with the substrate of glucose or sucrose, which can be employed in acetoin fermentation.

In this invention the strain *Bacillus pumilus* XH195 has been deposited in a microorganism deposit center in Germany (Deutsche Sammlung von Mikroorganismen und Zellkulturen GmbH, Braunschweig). The deposit number is DSM 16187.

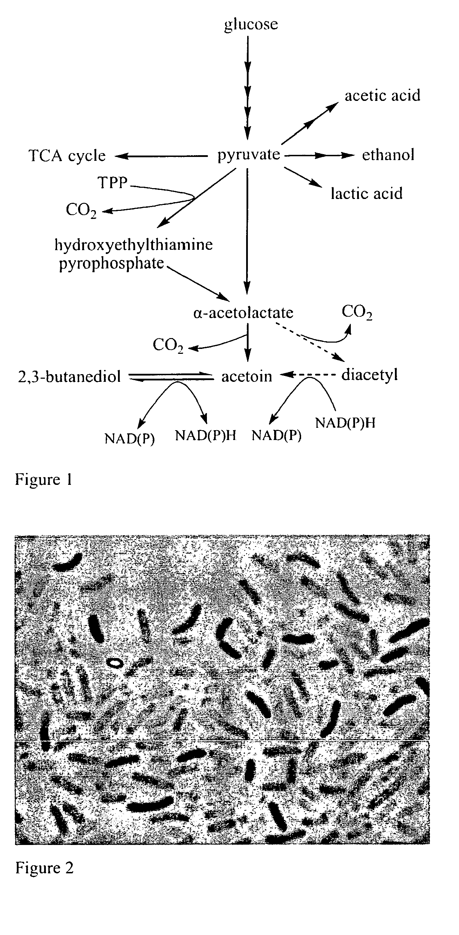

The strain *Bacillus pumilus* XH 195 DSM 16187 is rod-shaped, 1.5 µm to 3.0 µm in length and 0.6 µm to 0.7 µm in diameter (Figure 2). The colony color of the bacterial strain is yellow or white. The strain can produce spores and is positive in VP test. The strain is able to produce acid utilizing glucose, arabinose, xylose, or mannitol. This strain can hydrolyze casein, gelatin, and Tween 80. This strain can make use of citrate, grow in the media containing 100 g/L NaCl, and grow at 50°C. The physiological and biochemistry characteristics of this strain are described in the following table.

**Table Characteristics of the strain Bacillus pumilus XH195 DSM 16187**

| Characteristics of the strain | Results |
|---|---|
| Rods | + |
| Width | 0.6 ∼ 0.7µm |
| Length | 1.5 ∼ 3.0µm |
| Spores | + |
| Ellipsoid | + |
| Sporangium | - |
| VP reaction | + |
| pH in VP | 5.0 |
| Acid from glucose | + |
| Acid from arabinose | + |
| Acid from xylose | + |
| Acid from mannitol | + |
| Acid from fructose | + |
| Gas from glucose | - |
| Hydrolysis of casein | + |
| Hydrolysis of gelatin | + |
| Hydrolysis of starch | - |
| Hydrolysis of Tween 80 | + |
| Hydrolysis of esculine | - |
| Utilization of citrate | + |
| Utilization of propionate | - |
| Degradation of tyrosin | - |
| Phenylalanin deaminase | - |
| NO₃ to NO₂ | - |
| Indol | - |
| Growth at pH 5.7 | + |
| Growth with 2% NaCl | + |
| Growth with 5% NaCl | + |
| Growth with 7% NaCl | + |
| Growth with 10% NaCl | + |
| Growth at 45°C | + |
| Growth at 50°C | + |
| Growth at 55°C | - |
| Growth with 0.001% lysozym | + |
| Arginin dihydrolase | - |

The strain *Bacillus pumilus* XH 195 DSM 16187 has the typical fatty acid profile of *Bacillus.*

The 16S rDNA sequence of *Bacil*/*us pumilus* XH 195 DSM 16187 is 98.7% - 100% similar to other *Bacillus pumilus* strains.

The strain *Bacillus pumilus* XH195 DSM 16187 is able to grow in high osmotic LB medium (LB medium containing high Sugar, LBS).

One liter of LBS contained 200 g of glucose, 10g of peptone, 5 g of yeast extract, and 10g of NaCl. Twenty grams per liter of agar was added as to the solid LBS. The media were sterilized at 121°C for 15 min.

The *Bacillus pumilus* XH195 DSM 16187 is employed in acetoin fermentation.

When employing *Bacillus pumilus* XH195 DSM 16187 to produce acetoin with glucose fermentation medium or sucrose fermentation medium, the temperature is 30°C ∼ 40°C. The bacterium in conical flasks was incubated on a shaker at 160 ∼ 220 r/min for 40 ∼ 70 h to botain the mature acetoin fermentation broth.

The components of the above-mentioned glucose fermentation medium were as follows. One liter of distilled water contained 200 g of glucose, 50 g of NH₄Cl, 0.50 g of KH₂PO₄, 4.0 g of K₂HPO₄·3H₂O, 2.0 ml of 10 g/L of CaCl₂ solution, 2.0 ml of 100 g/L of MgCl₂·6H₂O solution, 200 µl of 10 g/L of FeCl₃ solution, 200 µl of 50 g/L of NaCl solution, 5.0 ml of 10 g/L of yeast extract solution, 5.0 ml of metal ions mixture solution, and 200 µl of vitamin mixture solution. The medium was sterilized at 121°C for 15 min.

Therein, the components of the metal ions mixture solution were as follows. One liter of distilled water contained 0.50 g of ZnCl₂, 0.50 g of FeCl₃, 0.50 g of MnCl₂·4H₂O, 0.10 g of NaMoO₄·2H₂O, 0.050 g of CuCl₂·2H₂O, 0.050 g of Na₂WO₄·2H₂O, and 120 mmol/L of HCl.

Therein, the components of the vitamin mixture solution were as follows. One liter of distilled water contained 0.40 g of calcium pantothenate, 0.20 g of inositol, 0.40 g of nicotinic acid, 0.40 g of VB₆, 0.20 g of *p*-aminobenzoic acid, and 0.5 mg of VB₁₂.

The above-mentioned sucrose fermentation medium was same as the glucose fermentation medium, only excepting that 200 g of glucose was replaced with 180 g of sucrose.

The strain of *Bacillus pumi*/*us* XH 195 DSM 16187 was inoculated in 50 ml of glucose fermentation medium in 300-ml conical flasks. The flasks were kept on a shaker at 180 r/min and 37°C. Then the concentration of acetoin in the broth reached 63.0 g/L at 60 h.

The strain of *Bacillus pumilus* XH 195 DSM 16187 was inoculated in 50 ml of sucrose fermentation medium in 300-ml conical flasks. The flasks were kept on a shaker at 180 r/min and 37°C. Then the concentration of acetoin in the broth reached 58.1 g/L at 60 h.

The acetoin product in the fermentation broth, which could be extracted using conventional methods, has optical activity.

The present invention overcomes the bottleneck of acetoin fermentation by providing the high-yield acetoin-producing strain *Bacillus pumilus* XH195 DSM 16187. All other strains as mentioned in BACKGROUND OF THE INVENTION have the shortcomings of low yield or producing acetoin as a by-product of 2,3-butanediol fermentation, and are difficult to achieve industrial-scale production.

The present invention provides an acetoin production approach by microbial fermentation, which has the advantages of low material costs, mild reaction conditions, high acetoin yield (63.0 g/L in flask fermentation), and simple product recovery. The product of acetoin is natural, optically active, low cost, and environmentally benign.

### DESCRIPTION OF THE DRAWINGS

Figure 1 illustrates the metabolic pathway of glucose to acetoin.
Figure 2 shows the electron microscopy image (2700 fold) of the strain *Bacillus pumilus* XH195 DSM 16187 in the present invention, which was performed in Deutsche Sammlung von Mikroorganismen und Zellkulturen.

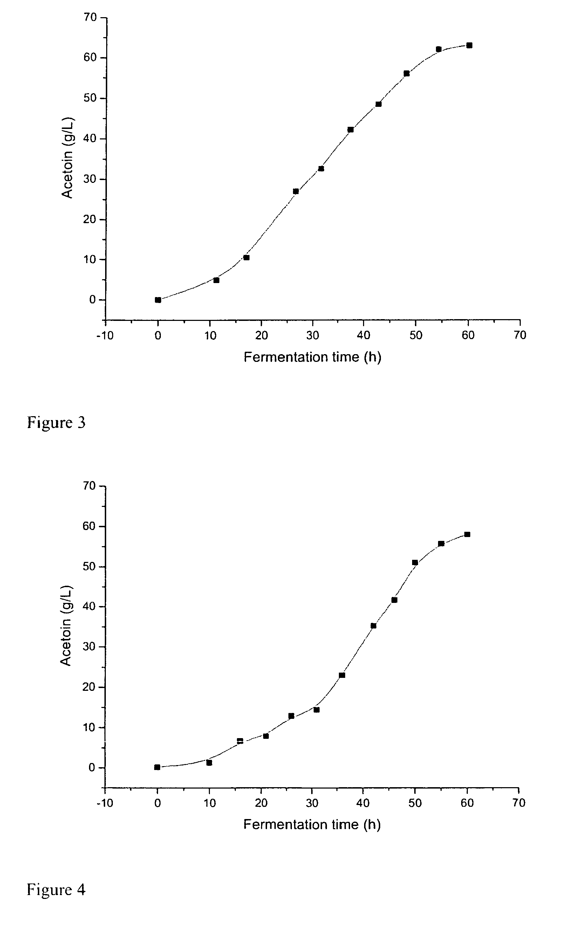

Figure 3 illustrates the time course of acetoin production by *Bacillus pumi*/*us* XH 195 DSM 16187 in glucose fermentation medium.
Figure 4 illustrates the time course of acetoin production by *Bacillus pumi*/*us* XH 195 DSM 16187 in sucrose fermentation medium.

### EXAMPLES

### EXAMPLE 1: screening of high sugar-tolerant strains for acetoin production

Soil samples obtained from apple groves and vine yards were soaked in LB medium containing high Sugar (LBS) for over 24 hours and 50 ml of the solution was incubated in a 300 ml conical flask at 37°C for 48 hours with shaking at 180 r/min. The culture was diluted 10² and 10³ fold, plated on solid LBS, and incubated for 24 hours at 37°C. Single colonies were isolated and cultured to detect the ability of acetoin production. Thus high sugar-tolerant acetoin-producing strains were selected. One liter of LBS contained 200 g of glucose, 10g of peptone, 5 g of yeast extract, and 10g of NaCl. Twenty grams per liter of agar was added as to the solid LBS. The media were sterilized at 121°C for 15 min.

### EXAMPLE 2: obtaining the invention strain by mutagenesis

A loop of the strain isolated in EXAMPLE 1 cultured on LBS slant was placed in a 300-ml conical flask containing 50 ml sterilized LBS and cultivated for 24 hours at 37°C with shaking at 180 r/min to obtain the cell broth.

The cell broth was centrifuged for 5 min at 3000 r/min and the supernatant was discarded. The precipitate was washed and resuspended with sterilized normal saline and the resulting suspension was transferred to a sterilized conical flask containing glass beads to scatter the cells. The suspension was then transferred to a filter with filter paper, and the unicellular filtrate was collected in test tubes for future use.

One milligram of N-methyl-N'-nitro-N-nitrosoguanidine (NTG) was dissolved in 2 ml of 0.1 mol/L phosphate buffer and 1 ml of the NTG solution was added to 1 ml of the above-prepared cell suspension. After shaking for 30 min at 37°C, the culture was diluted 1000 fold to stop the mutation and diluted 10², 10³, 10⁴, 10⁵ fold to plate on LBS plates. After incubation at 37°C for 36 hours, single colonies were isolated and cultured to detect the ability of acetoin production. The strain with the highest yield was selected as the invention strain.

The above-mentioned strain is rod in shape, 1.5 µm to 3.0 µm in length and 0.6 µm to 0.7 µm in diameter. The colony color of the bacterial strain is yellow or white. It can produce spores and the VP reaction is positive. It can utilize glucose, arabinose, xylose, mannitol, or fructose to form acids. The bacterial strain can also hydrolyze casein, gelatin and Tween 80, utilize citrate, grow in medium containing 100 g/L NaCl, and grow at 50°C. This strain has the typical fatty acid profile as *Bacillus* and its 16S rDNA sequence shares 98.7% ∼ 100% similarity with other *Bacillus pumilus* strains.

The present invention strain was named as *Bacillus pumilus* XH195 and it has been deposited in Deutsche Sammlung von Mikroorganismen und Zellkulturen on 27 January 2004. The deposit number is DSM 16187.

### EXAMPLE 3: preparation of the cell broth of Bacillus pumilus XH195 DSM 16187

A loop of the strain *Bacillus pumilus* XH 195 DSM 16187 cultured on LBS slant was inoculated in a 300-ml conical flask containing 50 ml sterilized LBS broth and cultivated at 37°C with shaking at 180 r/min for 24 hours to get the resulting cell broth.

### EXAMPLE 4: preparation of the mature acetoin fermentation broth using the glucose fermentation medium

The cell broth obtained according to EXAMPLE 3 was inoculated at a volume ratio of 50 ml per liter to 300-ml conical flasks each of which contained 50 ml of sterilized glucose fermentation medium. The bacterium was cultured at 37°C with shaking at 180 r/min. Sampling was made every other 4 hours and the concentration of acetoin was measured. When the concentration of acetoin reached 63.0 g/L at 60 h, the flasks were removed from the shaker to stop fermentation. So the mature acetoin fermentation broth was obtained.

The components of the above-mentioned glucose fermentation medium were as follows. One liter of distilled water contained 200 g of glucose, 50 g of NH₄Cl, 0.50 g of KH₂PO₄, 4.0 g of K₂HPO₄·3H₂O, 2.0 ml of 10 g/L of Cacl₂ solution, 2.0 ml of 100 g/L of MgCl₂·-6H₂O solution, 200 µl of 10 g/L of FeCl₃ solution, 200 µl of 50 g/L of NaCl solution, 5.0 ml of 10 g/L of yeast extract solution, 5.0 ml of metal ions mixture solution, and 200 µl of vitamin mixture solution. The medium was sterilized at 121°C for 15 min.

Therein, the components of the metal ions mixture solution were as follows. One liter of distilled water contained 0.50 g of ZnCl₂, 0.50 g of FeCl₃, 0.50 g of MnCh2·H₂O, 0.10 g of NaMoO₄·2H₂O, 0.050 g of CuCl₂·2H₂O, 0.050 g of Na₂WO₄·2H₂O, and 120 mmol/L of HCl.

Therein, the components of the vitamin mixture solution were as follows. One liter of distilled water contained 0.40 g of calcium pantothenate, 0.20 g of inositol, 0.40 g of nicotinic acid, 0.40 g of VB₆, 0.20 g of *p*-aminobenzoic acid, and 0.5 mg of VB₁₂.

### EXAMPLE 5: altering the fermentation conditions to obtain the mature acetoin fermentation broth with Bacillus pumilus XH 195 DSM 16187 using the glucose fermentation medium

EXAMPLE 4 was performed with altered fermentation temperature of 30°C and shaking speed of 220 r/min. The concentration of acetoin reached 53.2 g/L at 70 h.

### EXAMPLE 6: altering the fermentation conditions to obtain the mature acetoin fermentation broth with Bacillus pumilus XH195 DSM16187 using the glucose fermentation medium

EXAMPLE 4 was performed with altered fermentation temperature of 40°C and shaking speed of 160 r/min. The concentration of acetoin reached 55.7 g/L at 40 h.

### EXAMPLE 7: altering the fermentation conditions to obtain the mature acetoin fermentation broth with Bacillus pumilus XH 195 DSM 16187 using the sucrose fermentation medium

EXAMPLE 4 was performed with the sucrose fermentation medium instead of the glucose fermentation medium. The concentration of acetoin reached 58.1 g/L at 60 h.

The above-mentioned sucrose fermentation medium was same as the glucose fermentation medium of EXAMPLE 4, only excepting that 200 g of glucose was replaced with 180 g of sucrose.

### EXAMPLE 8: altering the fermentation conditions to obtain the mature acetoin fermentation broth with Bacillus pumilus XH 195 DSM 16187 using the sucrose fermentation medium

EXAMPLE 7 was performed with altered fermentation temperature of 33°C and shaking speed of 200 r/min. The concentration of acetoin reached 54.1 g/L at 68 h.

### EXAMPLE 9: altering the fermentation conditions to obtain the mature acetoin fermentation broth with Bacillus pumi/us XH 195 DSM 16187 using the sucrose fermentation medium

EXAMPLE 7 was performed with altered fermentation temperature of 40°C and shaking speed of 170 r/min. The concentration of acetoin reached 55.1 g/L at 45 h.

## Claims

1. Isolated bacterial strain for the production of high-yield of acetoin, **characterized in that** said isolated bacterial strain is a *Bacillus pumilus* XH195, deposited in Deutsche Sammlung von Mikroorganismen und Zellkulturen on January 27, 2004 and having as deposit number DSM 16187.

2. Isolated bacterial strain according to claim 1, being a rod-shaped bacterium strain, with a length of 1,5 to 3,0 µm and a diameter of 0,6 to 0,7 µm.

3. Isolated bacterial strain according to claim 1 or claim 2, having a colony color yellow or white.

4. Isolated bacterial strain according to anyone of the claims 1 to 3, having a capability of sporulation and presenting a positive VP reaction.

5. Isolated bacterial strain according to anyone of the claims 1 to 4, having a fatty acid profile typical of one of *Bacillus.*

6. Isolated bacterial strain according to claims 1 to 5, having a 16s rDNA sequence which shares a similarity of 98,7% to 100% with other *Bacillus pumilus* strains.

7. Method of producing high-yield of acetoin comprising the fermentation of a bacterial strain *Bacillus pumilus* XH195, deposited in Deutsche Sammlung von Mikroorganismen und Zellkulturen on January 27, 2004 and having as deposit number DSM 16187.

8. Method according to claim 7, wherein said fermentation is conducted in a glucose or sucrose fermentation medium.

9. Method according to claim 8, wherein said glucose fermentation medium contains, per liter distilled water, 200 g glucose, 50 g NH₄Cl, 0,5 g KH₂PO₄, 4 g K₂HPO₄, 2 ml of 1% (w/w) CaCl₂, 2 ml of 10% (w/w) MgCl₂·6H₂O, 200 µl of 1% (w/w) FeCl₃, 200 µl of 5% (w/w) NaCl, 5 ml of 1% (w/w) yeast extract, 200 µl vitamin mixture solution, and 5 µl metal ions mixture solution.

10. Method according to claim 8, wherein said sucrose fermentation medium contains 180 g of sucrose, 50 g of NH₄Cl, 0,5 g of KH₂PO₄ 4 g of K₂HPO₄, 2 ml of 1% (w/w) CaCl₂, 2 ml of 10% (w/w) MgCl₂·6H₂O, 200 µl of 1% (w/w) FeCl₃, 200 µl of 5% (w/w) NaCl, 5 ml of 1% (w/w) yeast extract, 200 µl of vitamin mixture solution, and 5 ml of metal ions mixture solution in 1 liter of distilled water.

11. Method according to claim 9 or claim 10, wherein said metal ions mixture solution contains, per liter distilled water, 0,5 g ZnCl₂, 0,5 g FeCl₂, 0.5 g MnCl₂·4H₂O, 0,1 g Na₂MoO₄·2H₂O, 0.05 g CuCl₂·2H₂O, 0,05 g Na₂WO₄ 2H₂O, 120 mmol/L of HCl.

12. Method according to anyone of the claims 9 to 11, wherein said vitamin mixture solution is composed of 400 mg of calcium pantothenate, 200 mg of inositol, 400 mg of niacin, 400 mg of pyridoxine hydrochloride, 200 mg of *p*-aminobenzoic acid, and 0,5 mg of cyanocobalamin in 1 liter of distilled water.

## Patentansprüche

1. Isolierter Bakterienstamm für die Produktion von Acetoin mit hoher Ausbeute, **dadurch gekennzeichnet, dass** es sich bei dem isolierten Bakterienstamm um einen *Bacillus pumilus* XH195 handelt, der am 27. Januar 2004 in der Deutschen Sammlung von Mikroorganismen und Zellkulturen hinterlegt wurde und die Depotnummer DSM 16187 hat.

2. Isolierter Bakterienstamm nach Anspruch 1, bei dem es sich um einen stabförmigen Bakterienstamm mit einer Länge von 1,5 bis 3,0 µm und einem Durchmesser von 0,6 bis 0,7 µm handelt.

3. Isolierter Bakterienstamm nach Anspruch 1 oder 2 mit gelber oder weißer Koloniefarbe.

4. Isolierter Bakterienstamm nach einem der Ansprüche 1 bis 3, der die Fähigkeit zur Sporenbildung besitzt und eine positive VP-Reaktion aufweist.

5. Isolierter Bakterienstamm nach einem der Ansprüche 1 bis 4, der ein für *Bacillus* typisches Fettsäureprofil aufweist.

6. Isolierter Bakterienstamm nach einem der Ansprüche 1 bis 5 mit einer 16s-rDNA-Sequenz, welche eine Ähnlichkeit von 98,7% bis 100% mit anderen *Bacillus-pumilus-Stämmen* aufweist.

7. Verfahren des Produzierens von Acetoin mit hoher Ausbeute, das die Fermentation eines Bakterienstammes *Bacillus pumilus* XH195 umfasst, welcher am 27. Januar 2004 in der Deutschen Sammlung von Mikroorganismen und Zellkulturen hinterlegt wurde und die Depotnummer DSM 16187 hat.

8. Verfahren nach Anspruch 7, wobei die Fermentation in einem Glukose- oder Saccharosefermentationsmedium durchgeführt wird.

9. Verfahren nach Anspruch 8, wobei das Glukosefermentationsmedium 200 g Glukose, 50 g NH₄Cl, 0,5 g KH₂PO₄, 4 g K₂HPO₄, 2 ml 1%iges (Gew./Gew.) CaCl₂, 2 ml 10%iges (Gew./Gew.) MgCl₂.6H₂O, 200 µl 1%iges (Gew./Gew.) FeCl₃, 200 µl 5%iges (Gew./Gew.) NaCl, 5 ml 1%igen (Gew./Gew.) Hefeextrakt, 200 µl Vitamingemischlösung und 5 µl Metallionengemischlösung pro Liter destilliertes Wasser enthält.

10. Verfahren nach Anspruch 8, wobei das Saccharosefermentationsmedium 180 g Saccharose, 50 g NH₄Cl, 0,5 g KH₂PO₄, 4 g K₂HPO₄, 2 ml 1%iges (Gew./Gew.) CaCl₂, 2 ml 10%iges (Gew./Gew.) MgCl₂.6H₂O, 200 µl 1 %iges (Gew./Gew.) FeCl₃, 200 µl 5%iges (Gew./Gew.) NaCl, 5 ml 1%igen (Gew./Gew.) Hefeextrakt, 200 µl Vitamingemischlösung und 5 ml Metallionengemischlösung in 1 Liter destilliertem Wasser enthält.

11. Verfahren nach Anspruch 9 oder Anspruch 10, wobei die Metallionengemischlösung 0,5 g ZnCl₂, 0,5 g FeCl₂, 0,5 g MnCl₂.4H₂O, 0,1 g Na₂MoO₄.2H₂O, 0,05 g CuCl₂.2H₂O, 0,05 g Na₂WO₄.2H₂O, 120 mmol/l HCl pro Liter destilliertes Wasser enthält.

12. Verfahren nach einem der Ansprüche 9 bis 11, wobei die Vitamingemischlösung aus 400 mg Calciumpantothenat, 200 mg Inositol, 400 mg Niacin, 400 mg Pyridoxinhydrochlorid, 200 mg *p-*Aminobenzoesäure und 0,5 mg Cyanocobalamin in 1 Liter destilliertem Wasser zusammengesetzt ist.

## Revendications

1. Souche bactérienne isolée pour la production d'un haut rendement d'acétoïne, **caractérisée en ce que** ladite souche bactérienne isolée est un Bacillus pumilus XH195, et a été déposée à la Deutsche Sammlung von Mikroorganismen und Zellkulturen (Collection allemande de microorganismes et de culture cellulaire) le 27 janvier 2004 et ayant comme numéro de dépôt DSM 16187.

2. Souche bactérienne isolée selon la revendication 1, étant une souche de bactéries en bâtonnet, d'une longueur de 1,5 à 3,0 µm et d'un diamètre de 0,6 à 0,7 µm.

3. Souche bactérienne isolée selon la revendication 1 ou la revendication 2, présentent une couleur de colonie jaune ou blanche.

4. Souche bactérienne isolée selon l'une quelconque des revendications 1 à 3, possédant une capacité de sporulation et présentant une réaction VP positive.

5. Souche bactérienne isolée selon l'une quelconque des revendications 1 à 4, possédant un profil d'acides gras typique d'un Bacillus.

6. Souche bactérienne isolée selon l'une quelconque des revendications 1 à 5, possédant une séquence d'ADNr 16s partageant une similitude de 98,7% à 100% avec d'autres souches de Bacillus pumilus.

7. Procédé de production à haut rendement d'acétoïne, comprenant la fermentation d'une souche bactérienne de Bacillus pumilus XH195, déposée à la Deutsche Sammlung von Mikroorganismen und Zellkulturen (Collection allemande de microorganismes et de culture cellulaire) le 27 janvier 2004 et ayant comme numéro de dépôt DSM 16187.

8. Procédé selon la revendication 7, dans lequel ladite fermentation est réalisée dans un milieu de fermentation à base de glucose ou de sucrose.

9. Procédé selon la revendication 8, dans lequel ledit milieu de fermentation de glucose contient , par litre d'eau distillée, 200 g de glucose, 50 g de NH₄Cl, 0,5 g de KH₂PO₄, 2 ml de CaCl₂ à 1% en poids, 2 ml de MgCl₂.6H₂O à 10% en poids, 200 µl de FeCl₃ à 1% en poids, 200 µl de NaCl à 5% en poids, 5 ml d'extrait de levure à 1% en poids, 200 µl d'une solution d'un mélange de vitamines, et 5 µl d'une solution d'un mélange d'ions métalliques.

10. Procédé selon la revendication 8, dans lequel ledit milieu de fermentation à base de sucrose contient 180 g de sucrose, 50 g de NH₄Cl, 0,5 g de KH₂PO₄, 4 g de K₂HPO₄, 2 ml de CaCl₂ à 1 % en poids, 2 ml de MgCl₂.6 H₂O à 10% en poids, 200 µl de FeCl₃ à 1% en poids, 200 µl de NaCl à 5% en poids, 5 ml d'extrait de levure à 1£ en poids, 200 µl d'une solution d'un mélange de vitamines et 5 µl d'une solution d'un mélange d'ions métalliques dans 1 l d'eau distillée.

11. Procédé selon la revendication 9 ou la revendication 10, dans lequel ladite solution d'un mélange d'ions métalliques contient, par litre d'eau distillée, 0,5 g de ZnCl₂, 0,5 g de FeCl₂, 0,5 g de MnCl₂.4H₂O, 0,1 g de Na₂MoO₄.2H₂O, 0,05 g de CuCl₂.2H₂O, 0,05 g de Na₂WO₄.2H₂O, 120 mmoles/l de HCl.

12. Procédé selon l'une quelconque des revendications 9 à 11, dans lequel ladite solution d'un mélange de vitamines est composé de 400 mg de pantothénate de calcium, 200 mg d'inositol, 400 mg de niacine, 400 mg de chlorhydrate de pyridoxine, 200 mg d'acide p-aminobenzoïque et 0,5 mg de cyanocobalamine dans 1 litre d'eau distillée.
